# EUROPEAN PATENT APPLICATION

(11) **EP 4 729 542 A1**
(43) Date of publication of application: **22.04.2026**
(21) Application number: 24207039.9
(22) Date of filing: 16.10.2024
(51) Int. Cl.: C07K 16/28, A61K 39/00, A61P 35/00

(54) **ANTIGEN-BINDING POLYPEPTIDES AGAINST CD229**

(71) Applicant: Universitätsklinikum Hamburg-Eppendorf, 20246 Hamburg (DE)
(72) Inventor: Nolte, Friedrich, 20246 Hamburg (DE); Bannas, Peter, 20246 Hamburg (DE); Eden, Thomas, 20246 Hamburg (DE); Schaffrath, Alessa Zoe, 20246 Hamburg (DE); Duttmann, Anya, 20246 Hamburg (DE); Hambach, Julia, 20246 Hamburg (DE); Menzel, Stephan, 20246 Hamburg (DE); Gebhardt, Anna Josephine, 20246 Hamburg (DE); Tode, Natalie, 20246 Hamburg (DE)
(74) Representative: Eisenführ Speiser

(57) **Abstract**

The invention relates to antigen-binding polypeptides comprising the CDR1, CDR2 and CDR3 region of a VHH domain of a camelid heavy chain antibody. The polypeptides bind specifically to CD229 and are therefore suitable for the diagnosis as well as for the therapeutic and prophylactic treatment of diseases characterised by increased CD229 expression. Fusion proteins, conjugates and pharmaceutical compositions comprising the antigen-binding polypeptides are also disclosed. In addition, the invention relates to the use of such antigen-binding polypeptides in methods for detecting CD229 or CD229-expressing cells in a biological sample. Methods for purifying and concentrating CD229 or CD229-expressing cells using the antigen-binding polypeptides are also described.

## Description

The invention relates to antigen-binding polypeptides comprising the CDR1, CDR2 and CDR3 region of a VHH domain of a camelid heavy chain antibody. The polypeptides bind specifically to CD229 and are therefore suitable for cancer and immunotherapy that benefit from the depletion of malignant or autoantibody-producing cells. In particular, the polypeptides are suitable for the diagnosis as well as for therapeutic and prophylactic treatment of diseases characterized by increased CD229 expression. Conjugates and pharmaceutical compositions comprising the antigen-binding polypeptides are also disclosed. In addition, the invention relates to the use of such antigen-binding polypeptides in methods for detecting CD229 or CD229-expressing cells in a biological sample. Methods for purifying and concentrating CD229 or CD229-expressing cells using the antigen-binding polypeptides are also described.

### BACKGROUND

The search for new therapeutic targets has led to the identification of immunosuppressive adenosine as an important regulator of antitumor immunity. Selective inhibitors targeting various components of the adenosinergic pathway have been contemplated for use in cancer therapy. A component of the adenosinergic pathway which is of particular interest in this respect is CD38. While CD38 inhibition by antibody targeting has been successfully applied, off-target effects are commonly reported. Accordingly, there is a need for improved strategies that allow more specific and effective tumour cell targeting. Simultaneous targeting of more than one antigen appears to be a promising approach, as a decrease of off-target effects and improvement of selective targeting of tumour cells can be expected. In this regard, CD229 constitutes a promising new therapeutic target.

CD38 is a transmembrane ecto-enzyme which catalyses the hydrolysis of nicotinamide adenine dinucleotide (NAD) to adenosine diphosphate ribose (ADPR). The human protein is predicted to include 300 amino acids. It encompasses a small intracellular domain, a helical transmembrane domain and a large extracellular domain. Expression of CD38 has been reported for plasma B cells, natural killer (NK) cells, B cells and T cells, as well as for a variety of other cell types. In addition, CD38 expression has been reported in relation to HIV infection, leukaemia, myelomas, solid tumours, type II diabetes mellitus and bone metabolism and multiple myeloma. Involvement of CD38 in the development and progression of these diseases was further supported by reports on the role of NAD in shaping the tumour environment and on the role of intermediates of the adenosinergic pathway in inflammatory processes. Accordingly, monoclonal antibodies (mAbs) targeting CD38 have been developed and used as therapeutic compounds (e.g., daratumumab, isatuximab). However, therapeutic approaches employing these conventional types of antibodies are characterized by low or inefficient cytotoxicity and off-target effects.

CD229 (LY9, SLAMF3) is a transmembrane protein which comprises four extracellular immunoglobulin domains (V1, C2, V3 and C4). In addition, CD229 comprises a single transmembrane domain and a C-terminal cytosolic domain containing two immunoreceptor tyrosine-based switch motifs (ITSM). The mature form of human CD229 consists of 655 amino acids. CD229 is expressed at high levels by healthy plasma cells and by malignant B cells, including multiple myeloma (MM) and marginal-zone lymphomas (MZL) and at moderate levels by other lymphomas, including chronic lymphocyte leukaemia (CLL), follicular lymphoma (FL), mantle-cell lymphoma (MCL) and diffuse large B cell lymphoma. CD229 thus is a potential therapeutic target for antibody-based tumour cell targeting.

Accordingly, CD229 and CD38 represent interesting targets for the diagnosis and therapy of various cancer diseases. Monoclonal antibodies against CD38, which can be used diagnostically and therapeutically, are known in the arts. However, the treatment of tumours with monoclonal antibodies is associated with considerable disadvantages. For example, monoclonal antibodies often show insufficient stability after administration into a patient. In addition, the costs involved in the production of monoclonal antibodies are high. Specifically, the production of humanised antibodies with decreased immunogenicity is laborious and cost-intensive. In addition, owing to their size, complete antibodies show limited tissue permeability.

In the state of the art, various strategies have been developed to solve the problems associated with the administration of antibodies. For example, fragments of full IgG antibodies are discussed for therapeutic purposes. However, these fragments often show reduced specificity and affinity with respect to the antigen compared to the complete antibody. Furthermore, these fragments often show only low solubility in aqueous solutions.

It has been described in the art that camelid antibodies have properties that render them particularly suitable for therapeutic use in humans. In addition to conventional antibodies, which each have two heavy and two light chains, camelids also produce antibodies that exclusively consist of heavy chains. These so-called heavy chain antibodies are homodimers of two identical heavy chains that interact with the antigen through a single variable domain called VHH (variable domain of the heavy chain of heavy chain antibodies).

The VHH domain of a camelid heavy chain antibody has three complementary determining regions (CDRs) and four framework regions (FRs) that alternate with the CDRs in the primary sequence of the VHH domain. The VHH domain of a heavy chain antibody hence forms a small polypeptide unit with high antigen-binding capacity. Due to an exceptionally long CDR3 region with a length of 7-25 amino acids, the VHH domain can bind in cavities of protein antigens, unlike conventional antibodies, and thus block the active site of an enzyme. VHH domains can be produced recombinantly as soluble proteins in bacteria or mammalian cells. Such recombinantly produced VHH domains are also called single-domain antibodies (sdABs) or nanobodies. Currently, several nanobodies are being tested in phase II clinical trials.

VHH single domain antibodies are more stable and about ten times smaller than conventional antibodies and therefore exert a high solubility and significantly improved tissue penetration properties. For this reason, VHH single domain antibodies have been proposed for the treatment of various diseases.

The present invention provides for the first time VHH single domain antibodies capable of specifically binding to CD229. Such VHH single domain antibodies directed against CD229 have not been known in the prior art. The single domain antibodies of the present invention are particularly suitable for targeting human plasma and multiple myeloma cells. The single domain antibodies of the present invention are further particularly suitable for immunotherapy that includes blocking of enzymes of the adenosinergic pathway. Such approaches preferably comprise simultaneous targeting of CD229 in combination with other highly expressed antigens on plasma and cancer cells (e.g. CD38, CD138 or CD269) to achieve selective targeting and efficient cell depletion. Preferably, CD229 and CD38 are simultaneously targeted.

### DESCRIPTION OF THE INVENTION

In the present invention, heavy chain antibodies were first generated by immunising alpacas with human or murine CD229. Subsequently, RNA was isolated from peripheral blood lymphocytes of the alpacas and converted into cDNA by reverse transcription. Based on the isolated RNA, the variable domains of the heavy chain antibodies were amplified by PCR. Phage display libraries were prepared by cloning the VHH fragments into a phagemid vector. These libraries were selected using CD229-transfected HEK cells in a panning procedure. The sequences of the selected single domain antibodies were then determined by sequencing and recombinantly expressed in HEK-6E cells. In this way, the present invention provides VHH single domain antibodies suitable for use in therapeutic and diagnostic methods. In addition, the present invention provides CDR regions from VHH single domain antibodies that can be used in the construction of recombinant antigen-binding polypeptides.

Accordingly, in a first aspect, the invention provides an antigen-binding polypeptide comprising the complementarity-determining regions CDR1, CDR2 and CDR3 of a VHH domain of a camelid heavy chain antibody necessary for binding of a VHH single domain antibody, wherein the polypeptide specifically binds to CD229.

The term "CD229" as used herein refers to the "cluster of differentiation protein 229". In a preferred embodiment, the protein to which the polypeptide of the invention binds is human CD229 (gene ID 4063). The amino acid sequence of isoform X1 of CD229 is set forth in SEQ ID NO: 137. In a particularly preferred embodiment, the polypeptide of the invention specifically binds to an epitope that is located in the extracellular domain, more preferably in one of the four immunoglobulin domains V1, C2, V3 and C4 of CD229. The extracellular domain of CD229 extends from amino acid 48 to amino acid 454.

The polypeptides according to the invention comprise CDR regions derived from heavy chain antibodies of camelids. Heavy chain antibodies are a particular group of immunoglobulins consisting exclusively of heavy chains. In addition to two constant domains, a heavy chain of such an antibody contains a single variable domain, which in turn consists of three CDR regions and four FR regions. Interaction with the antigen takes place exclusively via this variable domain, which is also referred to as the VHH domain. Due to distinctive loop structures in the CDR3, a VHH domain is also able to recognise cryptic epitopes. Heavy chain antibodies are produced in only a few species, e.g. in llamas and alpacas. The camelid group is a family of mammals from the order Artiodactyla (even-toed ungulates). The camelid family includes camels, dromedaries, llamas, alpacas, guanacos and vicuñas. In a particularly preferred embodiment of the invention, the antigen-binding polypeptide comprises CDR regions derived from a VHH domain of a llama or alpaca, and more preferably an alpaca.

The polypeptide according to the invention has a specific affinity for CD229, and more preferably human CD229. This means that a polypeptide according to the invention binds to CD229 with a binding affinity that is significantly higher than the binding affinity for binding to another protein that is not homologous to the sequence of CD229.

Preferably, the binding affinity of the polypeptide according to the invention is at least 2-fold, at least 5-fold, at least 10-fold, at least 20-fold, at least 50-fold, at least 75-fold, at least 100-fold, at least 150-fold, at least 200-fold, at least 250-fold, at least 500-fold, at least 600-fold, at least 700-fold, at least 800-fold, at least 900-fold, at least 1000-fold, at least 2000-fold, at least 5000-fold, or at least 10000-fold higher than the binding affinity for a protein not homologous to CD229, such as human albumin.

The binding affinity of the polypeptide according to the invention will preferably bind to its antigen with a dissociation constant (Kd) of 10⁻⁵ M or less, and more preferably in the range of 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less. Accordingly, the polypeptide according to the invention will preferably bind to its antigen with a dissociation constant (Kd) of 10⁻⁵ M to 10⁻¹² M, and more preferably 10⁻⁶ M to 10⁻¹² M, 10⁻⁷ M to 10⁻¹² M, 10⁻⁸ M to 10⁻¹² M, or 10⁻⁹ M to 10⁻¹² M. A Kd value of greater than 10⁻⁴ M is generally considered to indicate non-specific binding. Methods for measuring the binding affinities of antibodies are well known in the prior art and include, for example, the determination of Kd values by flow cytometry, surface plasmon resonance spectroscopy or microscale thermophoresis.

In a particularly preferred embodiment of the invention, the CD229-specific antigen-binding polypeptide specifically binds to the V1 domain of CD229. The V1 domain of CD229 extends from amino acid 48 to amino acid 158.

In a particularly preferred embodiment of the invention, the CD229-specific antigen-binding polypeptide comprises
(a) the CDR sequences shown in SEQ ID NO:1-3;
(b) the CDR sequences shown in SEQ ID NO:4-6;
(c) the CDR sequences shown in SEQ ID NO:7-9;
(d) the CDR sequences shown in SEQ ID NO:10-12;
(e) the CDR sequences shown in SEQ ID NO:13-15;
(f) the CDR sequences shown in SEQ ID NO:16-18;
(g) the CDR sequences shown in SEQ ID NO:19-21;
(h) the CDR sequences shown in SEQ ID NO:22-24;
(i) the CDR sequences shown in SEQ ID NO:25-27;
(j) the CDR sequences shown in SEQ ID NO:28-30;
(k) the CDR sequences shown in SEQ ID NO:31-33;
(l) the CDR sequences shown in SEQ ID NO:34-36;
(m) the CDR sequences shown in SEQ ID NO:37-39;
(n) the CDR sequences shown in SEQ ID NO:40-42;
(o) the CDR sequences shown in SEQ ID NO:43-45;
(p) the CDR sequences shown in SEQ ID NO:46-48;
(q) the CDR sequences shown in SEQ ID NO:49-51;
or CDR sequences which differ from the CDR sequences shown in (a)-(q) in not more than one amino acid per CDR region. It is preferred that CD229-specific antigen-binding polypeptide comprises either the CDR sequences shown in SEQ ID NO:1-3 or the CDR sequences shown in SEQ ID NO:4-6. It is hence preferred that the polypeptide comprises a CDR1 as shown in SEQ ID NO:1 or SEQ ID NO:4, i.e. a CDR consisting of the amino acid sequence Ile-Phe-Ile (IFI). Alternatively, it is preferred that the polypeptide comprises a CDR2 as shown in SEQ ID NO:2 or SEQ ID NO:5, i.e. a CDR consisting of the amino acid sequence Ser-Ser-Arg-Gly-Ser (SSRGS). It is particular preferred that the polypeptide comprises a CDR1 as shown in SEQ ID NO:1 or SEQ ID NO:4 and, in addition, a CDR2 as shown in SEQ ID NO:2 or SEQ ID NO:5.

With respect to the amino acid sequences set forth in SEQ ID NO:1 and SEQ ID NO:4 in the attached sequence listing, it is to be noted that only amino acids 1-3 (I-F-I) represent the CDR, while the amino acid M does not belong to the CDR. Similarly, it is to be noted with respect to the amino acid sequences set forth in SEQ ID NO:82 that only amino acids 1-3 (T-F-G) represent the CDR, while the amino acid I does not belong to the CDR.

In another particular preferred embodiment of the invention, the CD229-specific antigen-binding polypeptide specifically binds to the C2 domain of CD229. The C2 domain of CD229 extends from amino acid 159 to amino acid 235.

In another particularly preferred embodiment of the invention, the CD229-specific antigen-binding polypeptide comprises
(a) the CDR sequences shown in SEQ ID NO:52-54;
(b) the CDR sequences shown in SEQ ID NO:55-57;
(c) the CDR sequences shown in SEQ ID NO:58-60;
(d) the CDR sequences shown in SEQ ID NO:61-63;
(e) the CDR sequences shown in SEQ ID NO:64-66;
(f) the CDR sequences shown in SEQ ID NO:67-69;
or CDR sequences which differ from the CDR sequences shown in (a)-(f) in not more than one amino acid per CDR region.

In another particular preferred embodiment of the invention, the CD229-specific antigen-binding polypeptide specifically binds to the V3 domain of CD229. The V3 domain of CD229 extends from amino acid 251 to amino acid 363.

In another particularly preferred embodiment of the invention, the CD229-specific antigen-binding polypeptide comprises
(a) the CDR sequences shown in SEQ ID NO:70-72;
(b) the CDR sequences shown in SEQ ID NO:73-75;
(c) the CDR sequences shown in SEQ ID NO:76-78;
(d) the CDR sequences shown in SEQ ID NO:79-81,
(e) the CDR sequences shown in SEQ ID NO:82-84;
(f) the CDR sequences shown in SEQ ID NO:85-87;
or CDR sequences which differ from the CDR sequences shown in (a)-(f) in not more than one amino acid per CDR region.

In another particular preferred embodiment of the invention, the CD229-specific antigen-binding polypeptide specifically binds to the C4 domain of CD229. The C4 domain of CD229 extends from amino acid 364 to amino acid 452.

In another particularly preferred embodiment of the invention, the CD229-specific antigen-binding polypeptide comprises
(a) the CDR sequences shown in SEQ ID NO:88-90;
(b) the CDR sequences shown in SEQ ID NO:91-93;
(c) the CDR sequences shown in SEQ ID NO:94-96;
(d) the CDR sequences shown in SEQ ID NO:97-99,
(e) the CDR sequences shown in SEQ ID NO:100-102,
or CDR sequences which differ from the CDR sequences shown in (a)-(e) in not more than one amino acid per CDR region.

It is apparent to the skilled person that within the presently disclosed CDRs of SEQ ID NO:1-51, SEQ ID NO:52-69, SEQ ID NO:70-87 and SEQ ID NO:88-102, some modification of the amino acid sequence is possible without affecting the binding ability of the VHH single domain antibody. For example, it may be possible to modify CDR1 of the single domain antibody by substituting 1, 2, or 3 amino acids. Similarly, it may be possible to modify CDR2 of the single domain antibody by substituting 1 or 2 amino acids. Due to the length of CDR3, it may be possible to modify this CDR by substituting 1, 2, 3, 4, 5, or 6 amino acids.

In general, any of the amino acid residues within the CDRs shown in SEQ ID NO:1-51, SEQ ID NO:52-69, SEQ ID NO:70-87 and SEQ ID NO:88-102 may be replaced with another residue as long as the resulting CDR is still able (along with the other two CDRs) to specifically bind to CD229. Furthermore, 1, 2 or 3 amino acids within one of the sequences shown in SEQ ID NO:1-51, SEQ ID NO:52-69, SEQ ID NO:70-87 and SEQ ID NO:88-102 may also be deleted or added by addition as long as the ability to bind to CD229 is not completely eliminated.

Thus, also encompassed by the invention are antigen-binding polypeptides having CDRs that differ from the sequences shown in SEQ ID NO:1-51, SEQ ID NO:52-69, SEQ ID NO:70-87 and SEQ ID NO:88-102 in individual amino acid positions. The invention therefore specifically relates to antigen-binding polypeptides having CDR sequences that differ from any of the CDR sequences shown in SEQ ID NO:1-51, SEQ ID NO:52-69, SEQ ID NO:70-87 and SEQ ID NO:88-102 in not more than a single amino acid per CDR.

In a further preferred embodiment, the antigen-binding polypeptide of the invention comprises a complete VHH domain of a camelid heavy chain antibody. Such a VHH domain, when used alone, is capable of binding to the corresponding antigen and is therefore also referred to herein as a "VHH single domain antibody". In addition to the three CDR regions responsible for antigen binding, the VHH domain also comprises the four framework regions FR1-FR4, which are located between the CDR regions. The scaffold regions may be fully conserved in the VHH domain of the invention, or they may be in the form of fragments of the original scaffold sequences. For example, the scaffold sequences delimiting the VHH domain C-terminally and N-terminally may be truncated by one or more amino acids. Such modified VHH domains are expressly encompassed by the invention. As used herein, "VHH single domain antibody" means an antigen-binding polypeptide that lacks the constant domains CH2 or CH3 compared to a full heavy chain antibody. In a preferred embodiment, the antigen-binding polypeptide of the invention is a VHH single domain antibody.

Preferred antigen-binding polypeptides comprising a VHH domain of a camelid heavy chain antibody and specifically binding to CD229 comprise or consist of the sequences depicted in SEQ ID NO:103-119, SEQ ID NO:120-125, SEQ ID NO:126-131 and SEQ ID NO:132-136. The invention further extends to variants of the polypeptides shown in SEQ ID NO:103-119, SEQ ID NO:120-125, SEQ ID NO:126-131 and SEQ ID NO:132-136 that are homologous to the polypeptides shown in SEQ ID NO:103-119, SEQ ID NO:120-125, SEQ ID NO:126-131 and SEQ ID NO:132-136 and also have the ability to specifically bind CD229. As used herein, the term "variant" means an amino acid sequence that has been modified by deletion, substitution or addition of amino acids such that it has at least 80%, 85%, 90%, 91%, 92%, 93%, 94%, 95%, 96%, 97%, 98%, 99% sequence identity to any of the sequences shown in SEQ ID NO:103-119, SEQ ID NO:120-125, SEQ ID NO:126-131 and SEQ ID NO:132-136 when those sequences are optimally aligned, e.g., with the GAP or BESTFIT programs using the default gap procedure. Computer programs for determining amino acid sequence identity are sufficiently known in the prior art.

VHH single domain antibodies are distinguished from conventional mammalian antibodies in particular by the fact that only three CDRs are required for binding the antigen. The invention therefore also relates to those antigen-binding polypeptides which comprise only the CDR1, CDR2 and CDR3 regions required for binding CD229 of one of the polypeptides exemplified in SEQ ID NO:103-119, SEQ ID NO:120-125, SEQ ID NO:126-131 and SEQ ID NO:132-136, wherein the regions located between the CDR regions show only minor or essentially no similarity to the corresponding regions of one of the polypeptides shown in SEQ ID NO:103-119, SEQ ID NO:120-125, SEQ ID NO:126-131 and SEQ ID NO:132-136.

Thus, the framework regions (FR1-FR4) of the polypeptides provided herein can differ from that of the VHH single domain antibodies shown in SEQ ID NO:103-119, SEQ ID NO:120-125, SEQ ID NO:126-131 and SEQ ID NO:132-136 without adversely affecting the binding of the polypeptides. Preferably, the variants differ from the polypeptides shown in SEQ ID NO:103-119, SEQ ID NO:120-125, SEQ ID NO:126-131 and SEQ ID NO:132-136 only by the exchange of a few amino acids. Particularly preferably, the variants of the polypeptides shown in SEQ ID NO:103-119, SEQ ID NO:120-125, SEQ ID NO:126-131 and SEQ ID NO:132-136 differ from the polypeptides shown in SEQ ID NO:103-119, SEQ ID NO:120-125, SEQ ID NO:126-131 and SEQ ID NO:132-136 in less than 25, in less than 20, in less than 15, in less than 10, or in less than 5 amino acids. Preferably, variants of the polypeptides shown in SEQ ID NO:103-119, SEQ ID NO:120-125, SEQ ID NO:126-131 and SEQ ID NO:132-136 differ from the polypeptides shown in SEQ ID NO:103-119, SEQ ID NO:120-125, SEQ ID NO:126-131 and SEQ ID NO:132-136 in 5, 4, 3, 2 or only 1 amino acid position.

In one aspect, the invention thus relates to a CD229-specific antigen-binding polypeptide comprising an amino acid sequence selected from the group consisting of:
(a) the amino acid sequences of SEQ ID NO:103-119; and
(b) an amino acid sequence having at least 80% or more sequence identity to one of the amino acid sequences of SEQ ID NO:103-119.

In another aspect, the invention thus relates to a CD229-specific antigen-binding polypeptide comprising an amino acid sequence selected from the group consisting of:
(a) the amino acid sequences of SEQ ID NO:120-125; and
(b) an amino acid sequence having at least 80% or more sequence identity to one of the amino acid sequences of SEQ ID NO:120-125.

In another aspect, the invention thus relates to a CD229-specific antigen-binding polypeptide comprising an amino acid sequence selected from the group consisting of:
(a) the amino acid sequences of SEQ ID NO:126-131; and
(b) an amino acid sequence having at least 80% or more sequence identity to one of the amino acid sequences of SEQ ID NO:126-131.

In yet another aspect, the invention thus relates to a CD229-specific antigen-binding polypeptide comprising an amino acid sequence selected from the group consisting of:
(a) the amino acid sequences of SEQ ID NO:132-136; and
(b) an amino acid sequence having at least 80% or more sequence identity to one of the amino acid sequences of SEQ ID NO:132-136.

Preferably, the substitutions that distinguish the variants from the VHH single domain antibodies shown in SEQ ID NO:103-119, SEQ ID NO:120-125, SEQ ID NO:126-131 and SEQ ID NO:132-136 are conservative substitutions, i.e. substitutions of one or more amino acid residues by an amino acid of similar polarity acting as a functional equivalent. Preferably, the amino acid residue serving as a substitution is selected from the same group of amino acids as the amino acid residue to be substituted. For example, a hydrophobic residue may be replaced by another hydrophobic residue, or a polar residue may be replaced with another polar residue having the same charge. Functionally homologous amino acids which may be used for conservative substitution include, for example, non-polar amino acids such as glycine, valine, alanine, isoleucine, leucine, methionine, proline, phenylalanine, and tryptophan. Examples of uncharged polar amino acids include serine, threonine, glutamine, asparagine, tyrosine, and cysteine. Examples of charged polar (acidic) amino acids include histidine, arginine, and lysine. Examples of charged polar (basic) amino acids include aspartic acid and glutamic acid.

Furthermore, variants of the sequences shown in SEQ ID NO:103-119, SEQ ID NO:120-125, SEQ ID NO:126-131 and SEQ ID NO:132-136 also include polypeptides in which one or more amino acids have been inserted into the amino acid sequence of one of the polypeptides shown in SEQ ID NO:103-119, SEQ ID NO:120-125, SEQ ID NO:126-131 and SEQ ID NO:132-136. Such insertions can occur at any position of the polypeptides shown in SEQ ID NO:103-119, SEQ ID NO:120-125, SEQ ID NO:126-131 and SEQ ID NO:132-136, as long as the resulting variant of the polypeptide retains its immunological activity (i.e. the ability to specifically bind CD229). Furthermore, proteins shown in SEQ ID NO:103-119, SEQ ID NO:120-125, SEQ ID NO:126-131 and SEQ ID NO:132-136 in which one or more amino acids within the sequence are missing compared to the reference polypeptide are also considered variants of the polypeptide shown herein. Such deletions may affect any amino acid positions within the sequences shown in SEQ ID NO:103-119, SEQ ID NO:120-125, SEQ ID NO:126-131 and SEQ ID NO:132-136. In particular, the deletions may be those involving two or more (e.g. 3, 4 or 5) consecutive amino acids from any of the sequences of SEQ ID NO:103-119, SEQ ID NO:120-125, SEQ ID NO:126-131 and SEQ ID NO:132-136. Preferably, fewer than 25, fewer than 20, fewer than 15, fewer than 10, fewer than 5 amino acid positions in total have been inserted or deleted in the variant compared to the respective reference polypeptide having one of the sequences shown in SEQ ID NO:103-119, SEQ ID NO:120-125, SEQ ID NO:126-131 and SEQ ID NO:132-136.

Also included in the invention are immunologically active fragments of the polypeptides (or variants thereof) shown in SEQ ID NO:103-119, SEQ ID NO:120-125, SEQ ID NO:126-131 and SEQ ID NO:132-136. Immunologically active fragments are understood herein to be sequences which differ from the amino acid sequences shown in SEQ ID NO:103-119, SEQ ID NO:120-125, SEQ ID NO:126-131 and SEQ ID NO:132-136 or variants thereof by the absence of one or more amino acids at the N-terminus and/or at the C-terminus of the protein and which are still capable of specifically binding to CD229, preferably human CD229. For example, immunologically active fragments of the sequence shown in SEQ ID NO:103 may be fragments lacking the first two N-terminal and/or C-terminal amino acids of the polypeptide shown in SEQ ID NO:103. The skilled person will readily be able to determine the ability of fragments to bind to CD229, e.g. by competitive antibody assays.

The polypeptides shown in SEQ ID NO:103-119, SEQ ID NO:120-125, SEQ ID NO:126-131 and SEQ ID NO:132-136 and variants or fragments thereof may further be structurally modified at one or more positions in the amino acid sequence, such as by introducing a modified amino acid. According to the invention, these modified amino acids may be amino acids that have been modified by biotinylation, phosphorylation, glycosylation, acetylation, branching and/or cyclisation.

In some embodiments, it is preferred that the variants or fragments of the antigen-binding polypeptides of the invention retain more than 50% of the original binding affinity for CD229. It is even more preferred that the variants or fragments retain more than 60%, 70%, 80%, 90%, 95%, 96%, 97%, 98% or 99% of the original binding affinity of the antigen-binding polypeptides of the invention, e.g. the polypeptides shown in SEQ ID NO:103-119, SEQ ID NO:120-125, SEQ ID NO:126-131 and SEQ ID NO:132-136. The binding affinity for CD229 can be measured using *in vitro* assays. In other embodiments, it may be preferred that the variants or fragments of the antigen-binding polypeptides of the invention have a lower binding affinity of less than 50% of the original binding affinity for CD229. Such variants and fragments are preferably used in a multivalent targeting approach using e.g. a bispecific or multispecific polypeptide construct.

In another aspect, the invention provides a nucleic acid molecule encoding an antigen-binding polypeptide as defined above. The nucleic acid molecule may be DNA or RNA. Preferably, it is single-stranded or double-stranded DNA, such as genomic DNA or cDNA. Preferably, the polynucleotide encodes a polypeptide comprising one of the amino acid sequences shown in SEQ ID NO:103-119, SEQ ID NO:120-125, SEQ ID NO:126-131 and SEQ ID NO:132-136 or a variant of any of these.

The invention further comprises an expression vector comprising a nucleic acid molecule encoding an antigen-binding polypeptide of the present invention. Expression vectors are typically DNA constructs that have an origin of replication that allows the vector to replicate independently of the host cell. Expression vectors also include regulatory control elements that are functionally linked to the nucleotide sequences to be expressed and control their transcription and/or translation. Suitable control elements may be prokaryotic promoters (constitutive or regulatable, such as lac, trp, T3, T7 or gpt promoter), eukaryotic promoters (constitutive or regulatable, such as e.g. thymidine kinase promoter, SV40 promoter or CMV promoter), operators, transcription or translation enhancers, transcription or translation terminators, ribosome binding sites, polyadenylation signals, selection markers, silencers and repressor sequences. Numerous prokaryotic and eukaryotic expression vectors suitable for carrying out the present invention are known to those skilled in the art. Examples include the prokaryotic vectors pHEN2, pGEM, pBluescript and pUC, which are regularly used for heterologous expression in *E. coli.* Examples of eukaryotic expression vectors include pcDNA3.1, pOG44, pSV2CAT Rc/CMV, Rc/RSV, GEM1 and the like.

The invention also relates to a host cell comprising a nucleic acid molecule encoding an antigen-binding polypeptide of the present invention or an expression vector comprising such a nucleic acid molecule. In particular, it may be a prokaryotic or a eukaryotic host cell. Examples of host cells that may be used in the context of the invention include prokaryotic host cells, such as bacterial strains of *Escherichia coli, Bacillus subtilis* and the like. Suitable eukaryotic cells include yeast cells, insect cells, mammalian cells, plant cells and the like. The antigen-binding polypeptide of the present invention is preferably expressed in mammalian cells, e.g. HEK cells. The method of transforming or transfecting the host cell with an expression vector comprising a nucleic acid molecule encoding the antigen-binding polypeptide will depend on the type of expression vector and the host cell used. The skilled person will have no difficulty in selecting suitable vectors and host cells for recombinant expression.

The invention also provides a method for the recombinant production of an antigen-binding polypeptide of the invention, said method comprising: (a) culturing a host cell as described above under conditions that allow expression of a nucleic acid molecule encoding an antigen-binding polypeptide of the present invention; and (b) isolating the antigen-binding polypeptide from the culture. In the preparation of the antigen-binding polypeptides of the invention, the nucleic acid molecules, expression vectors and host cells described above may be used.

In another aspect, the invention relates to a method for detecting CD229 and/or CD229-expressing cells in a biological sample, comprising the steps of
(a) contacting a CD229-specific antigen-binding polypeptide described above and defined in the claims with the biological sample under conditions that allow the formation of complexes of the antigen-binding polypeptide and CD229; and
(b) detecting the complexes of the polypeptide and CD229/CD229-expressing cells.

The method comprises, as a first step, the contacting of an antigen-binding polypeptide of the invention with the biological sample. This step is carried out under conditions that allow for the formation of binding complexes consisting of the antigen-binding polypeptide and CD229 and/or CD229-expressing cells. Conditions that allow the formation of complexes consisting of the antigen-binding polypeptides of the invention and CD229 are known in the prior art and are sufficiently described in the context of binding of antibodies to protein antigens. Such conditions may include a temperature in the range of 4-37°C, a pH in the range of 6-8 and an incubation time in the range of several minutes to several hours. It will be apparent to one skilled in the art that suitable binding conditions for a given pair of an antigen-binding polypeptide and CD229 will depend on various factors, such as the concentration of the antigen-binding polypeptide, the anticipated concentration of CD229 in the sample, and the like. The skilled person will be able to determine suitable conditions for binding between polypeptide and CD229 based on routine experiments to optimise the formation of the binding complexes.

After the antigen-binding polypeptide has bound to CD229 and/or CD229-expressing cells, the immune complexes consisting of CD229 or CD229-expressing cells and the polypeptide specifically bound to them are detected. For this purpose, well-known methods can be used. For example, the antigen-binding polypeptide according to the invention can be provided with a detectable label prior to contacting the sample suspected of containing the CD229 and/or CD229-expressing cells. The label may be, for example, a fluorescent, chemiluminescent or radioactive label. Labelling with enzymes, such as horseradish peroxidase or alkaline phosphatase, is also possible. Another method that allows detection of the complexes is the recombinant expression of the antigen-binding polypeptides as fusion proteins that have one or more peptide epitopes that can be visualised using labelled antibodies.

In a particularly preferred embodiment of the invention, the method is used for the quantitative detection of CD229 and/or CD229-expressing cells in a biological sample. The quantitative detection can be carried out by methods that have been described in the prior art, for example by quantitative determination of a fluorescent, chemiluminescent or radioactive labelling of polypeptides in complexes. The determination can be carried out by comparison with a reference sample, i.e. by comparison with a sample containing a known amount of CD229 and/or CD229-expressing cells.

In a further aspect, the invention relates to a method for purifying and/or concentrating CD229 and/or CD229-expressing cells, comprising the steps of
(a) contacting a CD229-specific antigen-binding polypeptide as described above and defined in the claims with the biological sample under conditions that allow the formation of complexes of the antigen-binding polypeptide and CD229; and
(b) separating the complexes of the CD229-specific antigen-binding polypeptide and CD229/CD229-expressing cells from the sample.

Preferably, the CD229-specific polypeptide used in the above method is one comprising one or more of the CDR regions listed in SEQ ID NO:1-102 or modifications thereof as described above. In a particularly preferred embodiment, the CD229-specific polypeptides comprise the sequences or fragments listed in SEQ ID NO:1-102 or immunologically active fragments or variants thereof as described above.

Purification and/or concentration of CD229 and/or the CD229-expressing cells is preferably performed with antigen-binding polypeptides immobilised on a solid support, such as a material suitable for use in chromatographic separation procedures. Suitable column materials include Sepharose (e.g. Q-Sepharose, DEAE-Sepharose, SP-Sepharose) and the like. The CD229-binding polypeptides may further be immobilised on beads, e.g., agarose or glass beads. Preferably, the beads are magnetisable which facilitates separation of the binding complexes adhering to the beads after incubation in CD229-containing samples. Methods for coupling polypeptides to a solid support are sufficiently known in the prior art. For example, a coupling method may comprise the immobilisation of polypeptides which linked to biotin or derivatives of biotin to a support material that has been previously coated with streptavidin or derivatives thereof. Such compounds are commercially available from various suppliers. Alternatively, the protein may be directly bound to the support material by chemical reaction. The sample containing the CD229 and/or CD229-expressing cells is passed over the support material so that the CD229 and/or CD229-expressing cells are bound by the immobilised antigen-binding polypeptides. By using a suitable wash buffer, all non-specifically bound molecules and cells are washed from the support material while the CD229 and/or CD229-expressing cells are retained by the support material. In a final step, CD229 can be eluted from the material using a suitable elution buffer (e.g. a high salt buffer), thus obtaining the purified and/or concentrated CD229 protein. Alternatively, the CD229 bound to the support material or the CD229-expressing cells can be detected by an immunological detection method.

In some embodiments, the antigen-binding polypeptides are not coupled to a support. In these embodiments, separation from the sample can be achieved, e.g. by designing the CD229-specific antigen-binding polypeptide of the invention as a fusion polypeptide that comprises an affinity tag. Such fusion polypeptides can be efficiently purified using a material that has a particularly high affinity for the affinity tag used. For example, the affinity tag may have 6-12 histidine residues that specifically interact with a chelating nickel ion matrix. Alternatively, the affinity tag may comprise a glutathione S-transferase that can be purified using a glutathione matrix. Numerous other affinity tags are known in the art and can be used in conjunction with the antigen-binding polypeptides of the invention. Examples of affinity tag and affinity ligand pairs include maltose-binding protein (MBP) and maltose; avidin and biotin; streptag and streptavin or neutravidin.

Fusion proteins having an affinity tag may be produced, for example, by ligation of a DNA encoding an antigen-binding polypeptide of the invention with a sequence encoding the affinity tag. In cases where the affinity tag is not a peptide, the affinity tag may be conjugated to the antigen-binding polypeptide by chemical coupling reactions. For example, the antigen-binding polypeptide may be chemically coupled to biotin. Labelling with biotin can also be achieved by attaching to the antigen-binding polypeptide a peptide that is recognised by a biotin-protein ligase (EC 6.3.4.15), such as BirA from *E. coli.* Suitable peptides include the 13 amino acid minimal biotinylation sequence of the biotin carboxy carrier protein (BCCP), the 15 amino acid variant thereof called AviTag^{™}, and the 76 amino acid BioEase^{™} sequence, all of which are biotinylated by the biotin protein ligase BirA at a central lysine residue. Streptags such as Streptag II and One-STrEP-tag (IBA BioTAGnology, IBA GmbH, Germany) may also be used.

Furthermore, the antigen-binding polypeptides of the present invention can also be coupled with enzymes (such as alkaline phosphatase) or with recognition sequences for enzymes (such as the above BirA biotinylation sequences). Coupling of different antigen-binding polypeptides, such as VHHs directed against different epitopes of CD229, is also possible to enhance the efficiency of binding to the target antigen. For example, two or more of the single domain VHH antibodies described above may be coupled to biotin via a BirA recognition sequence and subsequently bound to avidin or streptavidin, or to modified streptavidin such as neutravidin or streptactin, via the high affinity binding of biotin. Avidin and streptavidin are tetrameric molecules that can bind four molecules of D-biotin. Such multimerised, tetravalent VHH single domain antibodies have an increased affinity for CD229. Streptavidin-coated beads, e.g. magnetic or Sepharose beads, can be used for multimerisation. Suitable beads are e.g. Strep-Tactin Magnetic Nanobeads (IBA BioTAGnology, IBA GmbH, Germany). The polypeptides of the present invention, and in particular the VHH single domain antibodies can of course also be coupled to immunoglobulins, e.g. to the Fc region of an immunoglobulin. In addition, the VHH single domain antibodies of the present invention can be prepared in the form of fusion proteins that comprise more than one VHH domain that binds to CD229, such as 2, 3, 4 or 5 VHH domains, which are expressed as a single protein chain. The additional VHH domains of the fusion protein may bind to the same or to different epitopes of CD229. Alternatively, the additional VHH domains of the fusion protein may bind to other target molecules, e.g. to CD38, CD269 or CD138, or to immune checkpoint proteins such as PD-1, PD-L1, CTLA-4, LAG3, TIM3, TIGIT, PVR-IG, PVR or PVR-L2. In a particular preferred embodiment, the invention relates to a fusion protein that comprises a first VHH directed against CD229 and a second VHH directed against CD38.

In a particularly preferred aspect of the invention, the CD229-specific polypeptides described above, preferably those comprising one or more of the CDR regions listed in SEQ ID NO:1-102 or modifications thereof as described above, and those comprising the sequences listed in SEQ ID NO: 103-136 or immunologically active fragments or variants thereof as described above, are used for therapeutic and diagnostic purposes.

The antigen-binding polypeptides of the present invention are particularly suitable for use in a method of diagnosing a disease characterised by increased expression of CD229. The diagnosis is based on the detection of cells or tissues that express increased levels of CD229. These cells and tissues can be detected via a suitable label after binding one of the polypeptides according to the invention.

Thus, the present invention also relates to a method of diagnosing a disease characterised by increased cellular expression of CD229, said method comprising
(a) contacting a CD229-specific antigen-binding polypeptide as described above and defined in the claims with a biological sample from a patient under conditions that allow the formation of complexes of the antigen-binding polypeptide and CD229; and
(b) detecting the complexes of the polypeptide and CD229/CD229-expressing cells;
wherein detection of the complexes in step (b) indicates that the patient has a disease characterised by increased CD229 expression.

The disease to be diagnosed is preferably a hyperproliferative disease. It is particularly preferred that the disease is multiple myeloma (MM) and marginal-zone lymphomas (MZL), chronic lymphocyte leukemia (CLL), follicular lymphoma (FL), mantle-cell lymphoma (MCL) and diffuse large B cell lymphoma.

The biological sample may be a tissue sample from a patient, e.g. a tissue sample from a tumor. It may also be a sample of a body fluid, such as blood or lymph, or a bone marrow sample. For example, the sample may be a blood sample containing B lymphocytes. Depending on the disease to be diagnosed, it may also be a specific fraction of a body fluid. For example, in a procedure used to diagnose MM, the fraction of the patient's blood containing the soluble extracellular domain of CD229 will be analysed.

The number of complexes detected in step (b) may be compared in the course of diagnosis with a reference sample analysed under identical conditions. The reference sample may be from a patient who has already been diagnosed with the disease to be detected, e.g. MM, MLZ, CLL, FL or MCL. In this case, a measurement indicating an approximately identical number of complexes in the reference sample and the patient sample indicates that the patient is also likely to be afflicted with the disease. Alternatively, the reference sample may be a sample from a healthy individual. In this case, the disease can be detected in the patient if the measured number of complexes in the patient sample is significantly higher compared to the reference sample.

In another embodiment, the present invention relates to a method for visualizing CD229-expressing cells or tissues in a subject, comprising the step of
(a) administering to the subject a CD229-specific antigen-binding polypeptide as described above and defined in the claims, wherein the polypeptide is provided with a detectable label; and
(b) visualizing the CD229-expressing cells or tissues in the body by detecting the label.

The above method enables a so-called in vivo imaging of CD229-expressing cells or tissue in a subject, such as a human. The polypeptides administered to the subject are provided with a label that allows detection of the label without explantation of the CD229-expressing cells or tissues. Preferably, the label is a fluorescent label, such as an Alexa dye. Suitable Alexa dyes include, but are not limited to, Alexa-647 and Alexa-680. The amount of labelled polypeptide required for visualisation depends on various factors, such as the label as well as the weight of the subject to whom the polypeptide is administered. Quantities can generally be used as described below in the context of therapeutic treatment.

Nicotine amide dinucleotide (NAD) and its metabolites are important substrates of extracellular membrane-bound enzymes such as CD296, CD38, CD203 and CD73. NAD is metabolized by CD38 to adenosine diphosphate-ribose (ADPR), which is further hydrolysed by CD203 and CD73 to adenosine monophosphate (AMP) and adenosine (ADO). It has been reported that signalling by the above substrates is involved in the immune response against pathogens, autoimmunity and inflammatory reactions. In addition, involvement of extracellular nucleotides in shaping the microenvironment of tumours has been reported. Accordingly, CD38 which is known to be expressed on tumour cells constitutes a promising candidate for targeted tumour therapy. It thus appears particularly useful to simultaneously target CD229 with other highly expressed antigens on malignant plasma cells such as CD38 to achieve selective targeting and efficient depletion of tumour cells.

Thus, another aspect of the present invention pertains to the antigen-binding polypeptides of the present invention for use in a method of treating a disease or disorder which is characterized by a pathologically enhanced level of CD229 activity. In a preferred embodiment, the disease to be treated is multiple myeloma (MM), marginal-zone lymphomas (MZL), chronic lymphocyte leukaemia (CLL), follicular lymphoma (FL), mantle-cell lymphoma (MCL) and diffuse large B cell lymphoma. In another preferred embodiment of the present invention, the tumour cells to be treated overexpresses CD229. In an even more preferred embodiment, the tumour to be treated overexpresses both CD229 and CD38.

The therapeutic effect of the polypeptides may be provided by killing CD229-expressing cells, in particular CD229-expressing tumour cells. For example, the killing of cells may result from antibody-dependent cell-mediated cytotoxicity (ADCC), complement-mediated cytotoxicity (CDC) and/or apoptotic processes which are induced by binding of the polypeptides of the invention to CD229. Alternatively, the polypeptides of the invention may be conjugated to one or more cytotoxic agents that result in cell death.

Preferably, binding of the antigen-binding polypeptide of the invention to CD229 leads to the death of at least 10%, 20%, 30%, 40%, 50%, 60%, 70%, 80% or even 90% or more of the CD229-expressing tumour cells of a population.

The CD229-specific antigen-binding polypeptides described above may be used in combination with other active ingredients, such as checkpoints inhibitors. Immune checkpoints, such as the cytotoxic T-lymphocyte-associated antigen 4 (CTLA-4) and programmed death 1 (PD-1), are negative regulators of the T-cell immune function. Inhibition of these checkpoints results in activation of the immune system and therefore has become a valuable immunotherapeutic approach for treating different types of cancer. It has been reported that during the treatment of cancer with checkpoint inhibitors, CD229 expression on T cells is increased as an escape mechanism. Accordingly, the administration of the CD229-specific antigen-binding polypeptides of the invention to cancer patient which are treated with checkpoint inhibitors is highly effective. Preferably, the CD229-specific antigen-binding polypeptides described above is used in combination with a checkpoint inhibitor selected from the group consisting of an inhibitor of CTLA-4, an inhibitor of PD-1, an inhibitor of CD274 (programmed cell death 1 ligand 1, PD-L1), an inhibitor of T cell immunoglobulin and ITIM domain (TIGIT), an inhibitor of poliovirus receptor-related immunoglobulin domain-containing (PVRIG), an inhibitor of poliovirus receptor (PVR), an inhibitor of poliovirus receptor-related 2 (PVRL2), and an inhibitor of T-cell immunoglobulin and mucin domain 3 (Tim-3).

The CD229-specific antigen-binding polypeptides disclosed herein may be used in combination with chemotherapy. Specifically, the polypeptides may be used in combination with one or more commonly known chemotherapeutic agents. Chemotherapeutic or cytotoxic agents which is suitable for being co-administered with the polypeptides of the present inventions include, but are not limited to, actinomycin, amsacrine, aminopterin, anthracycline, 5-azacytidine, 6-azauridine, azathioprine, bendamustine, biricodar, bleomycin, bortezomib, bryostatin, busulfan, calyculin, capecitabine, carmofur, carboplatin, chlorambucil, cisplatin, cladribine, clofarabine, cytarabine, dacarbazine, dasatinib, daunorubicin, decitabine, discodermolide, docetaxel, doxorubicin, doxifluridine, epirubicin, eribulin, estramustine, etoposide, exatecan, floxuridine, fludarabine, fluorouracil, 5-FU, fotemustine, gemcitabine, hydroxyurea, idarubicin, ifosfamide, imiquimod, irinotecan, irofulven, ixabepilone, lapatinib, lenalidomide, lomustine, lurtotecan, mafosfamide, masoprocol, mechlorethamine, melphalan, mercaptopurine, mitomycin, mitoxantrone, nelarabine, nilotinib, oxaliplatin, oxazaphosphorine, paclitaxel, pentostatin, pixantrone, plicamycin, procarbazine, raltitrexed, rebeccamycin, rubitecan, salinosporamide A, satraplatin, swainsonine, tariquidar, taxane, tegafur-uracil, temozolomide, testolactone, thiotepa, thioguanine, topotecan, trabectedin, tretinoin, triplatin, tetranitrate, and derivatives, tautomers and pharmaceutically active salts of the above compounds.

The CD229-specific antigen-binding polypeptides disclosed herein may be used in combination with radiotherapy. As used herein, radiation therapy or radiotherapy refers to the use of ionizing radiation for the targeted destruction of cancer cells or tissues. Radiation therapy includes various techniques for irradiating cancer tissue in a subject with high-energy radiation to destroy the cancer tissue. In the context of the invention, radiation therapy can include photon-based radiation, such as x-rays and gamma rays, and/or particle radiation, such as electron, carbon or proton beams. Preferably, radiation is performed as an external beam radiation therapy. The dose of radiation to be used in combination with the CD229-specific antigen-binding polypeptides of the invention will depend on the type of tumor to be treated and will normally be in a range of 20-75 Gy, preferably 30-60 Gy and more preferably 45-60 Gy. In a preferred aspect, the daily dose of radiation that is administered is 1.5-1.8 Gy for a child or adolescent and 2.0-2.5 Gy for an adult. Radiation therapy can be applied either before, simultaneously with, or after administration of the CD229-specific antigen-binding polypeptide.

In one embodiment, the CD229-specific antigen-binding polypeptides described above are used in combination with other antigen-binding polypeptides which are specific for other structures expressed on the surface of CD229-expressing cells. The binding of CD229-specific and other specific polypeptides to the cell may lead to a particularly efficient therapeutic effect, e.g. reduction of tumour cells. For example, the CD229-specific antigen-binding polypeptides may be used in combination with CD38-specific and/or CD203a-specific antigen-binding polypeptides, such as VHHs which are directed against CD38. Further, the CD229-specific antigen-binding polypeptides may be used in combination with other compounds that block or target other tumour markers, such as CD138, CD269, CD73 to induce ADCC and/or CDC.

Preferably, the antigen-binding polypeptide of the present invention will have a size that results in physicochemical properties suitable for such therapeutic application, e.g. good solubility and tissue penetration, thermal stability and similar properties. Preferably, the antigen-binding polypeptide of the present invention used for therapeutic treatment of a patient will have a size of 80-250 amino acids, wherein a size of 90-200 amino acids, 90-180 amino acids, 90-150 amino acids, 100-140 amino acids and 100-130 amino acids is preferred.

In a further aspect, the invention provides a method for treating a disease or disorder which is characterized by a pathologically enhanced level of CD229 activity. The treatment comprises administering one or more of the polypeptides of the invention to a patient which suffers from one of the diseases mentioned above, in particular cancer or a chronic disease. Such therapeutic treatment includes both treatment of an acute disease state and prophylactic treatment to prevent disease.

The CD229-binding polypeptide will preferably be administered in the form of a pharmaceutical composition. Accordingly, the invention also provides a pharmaceutical composition comprising an antigen-binding polypeptide of the present invention. The antigen-binding polypeptide is thereby administered to the patient in a therapeutically effective amount, i.e. in an amount sufficient to significantly reduce the number of CD229-expressing cells. In a preferred embodiment of the invention, the antigen-binding polypeptide is administered in an amount that results in a substantial reduction of CD229-expressing cells. The reduction of CD229-expressing cells may be local, e.g. in a tumour. The reduction of cells will preferably give rise to an improvement of one or more symptoms of the disease.

The exact amount of polypeptide that needs to be administered to achieve a therapeutic effect depends on several parameters. Factors relevant to the determination of the amount of polypeptide to be administered include, for example, the route of administration of the polypeptide, the size of the polypeptide in question, the nature of the cytotoxic residue (if any), the nature and severity of the disease, the medical history of the patient to be treated, and the age, weight, height and health status of the patient to be treated. A therapeutically effective amount of the antigen-binding polypeptide can be readily determined by one skilled in the art based on the common knowledge and the present disclosure.

The polypeptide is preferably administered to a subject in an amount sufficient to achieve a plasma concentration of from about 0.05 µg/ml to about 150 µg/ml, preferably from about 0.1 to about 50 µg/ml, more preferably from about 1 µg/ml to about 20 µg/ml, and typically from about 1 µg/ml to about 10 µg/ml, e.g. 5 µg/ml. The weekly dose may range from about 1 mg to about 500 mg of polypeptide per m² of body surface area of the patient. The weekly dose of the polypeptide may range from about 10-300 mg/m², preferably from about 100-200 mg/m², such as 150 mg/m². Depending on the size of the polypeptide, the amount of polypeptide to be administered can be adjusted.

The antigen-binding polypeptide of the present invention may be formulated for various modes of administration, e.g. oral administration as a tablet, capsule, powder, liquid or the like. However, it is preferred that the antigen-binding polypeptide is administered parenterally by intravenous injection or intravenous infusion. For example, administration may be by intravenous infusion, e.g. within 60 minutes, within 30 minutes, or within 15 minutes. It is further preferred that the antigen-binding polypeptide is administered locally by injection during surgery. Compositions suitable for administration by injection and/or infusion typically comprise solutions and dispersions, and powders from which such solutions and dispersions can be prepared. Such compositions will comprise the immunologically active polypeptide and at least one suitable pharmaceutically acceptable carrier. Suitable pharmaceutically acceptable carriers for intravenous administration include bacteriostatic water, Ringer's solution, physiological saline, phosphate buffered saline (PBS) and Cremophor EL^{™}. Sterile compositions for injection and/or infusion can be prepared by introducing the polypeptide in the required amount in a suitable carrier and then filtering it sterilely using a filter. Compositions for administration by injection or infusion should remain stable under storage conditions for a prolonged period after their preparation. The compositions may contain a preservative for this purpose. Suitable preservatives include chlorobutanol, phenol, ascorbic acid and thiomersal.

In one embodiment, the antigen-binding polypeptide of the present invention is formulated with carriers that protect the polypeptide from being excreted too rapidly from the body (sustained release formulations). Such formulations may include biocompatible polymers, such as ethylene vinyl acetate, polyanhydrides, polyglycolic acids, collagen, polyorthoesters and polylactic acids. Processes for the preparation of sustained-release formulations are sufficiently known in the prior art. These formulations may be provided in the form of semi-permeable films of hydrophobic polymers, e.g. in the form of microcapsules. The production of corresponding dosage forms as well as suitable excipients is described, for example, in Remington (2005).

In order to induce killing of CD229 expressing target cells by using the antigen-binding polypeptides of the present invention, it may be advantageous to conjugate one or more cytotoxic agents to the antigen-binding polypeptides described herein.

The invention thus also provides a conjugate comprising as a first component an antigen-binding polypeptide as described above, preferably a polypeptide comprising one or more of the CDR regions listed in SEQ ID NO:1-102 or modifications thereof as described above, or a polypeptide comprising one of the sequences listed in SEQ ID NO: 103-136 or immunologically active fragments or variants thereof as described above, and as a further component one or more cytotoxic residues. The coupling of the CD229-specific polypeptides to the cytotoxic agent can be carried out according to procedures customary in the state of the art.

The antigen-binding polypeptide of the present invention can be used in a clinical setting, e.g. for therapeutic or diagnostic purposes, or for research purposes. In one embodiment, the antigen-binding polypeptide of the present invention can be used in immunological and biochemical assays, including microscopy and flow cytometry of living or mildly fixed cells, immunoprecipitation and affinity purification of a target protein, ELISA of biological samples containing antigens, co-crystallization with a target antigen for 3D structure analyses, cryoelectron microscopy analyses of a target protein, and blockade or potentiation of the function of a target protein, such as its enzymatic or channel activity or its binding to specific ligands. In a particular preferred embodiment, the antigen-binding polypeptides of the present invention are used in the form of chimeric antibodies, chimeric antigen-receptors (CARs), bispecific T cell engagers (BiTEs), bispecific killer cell engagers (BiKEs) and nanobody-displaying adeno-associated viruses (AAVs). The above forms of the antigen-binding polypeptides of the present invention may also be constructed as being specific for one, two or multiple antigens and are thus mono-, bi- or multispecific.

Finally, the present invention also provides a kit for the detection of CD229 and CD229-expressing cells, the kit comprising an antigen-binding polypeptide as described above. The kit may further comprise other reagents and means necessary for the detection, e.g. reagents for visualising a label.

### BRIEF DESCRIPTION OF THE FIGURES

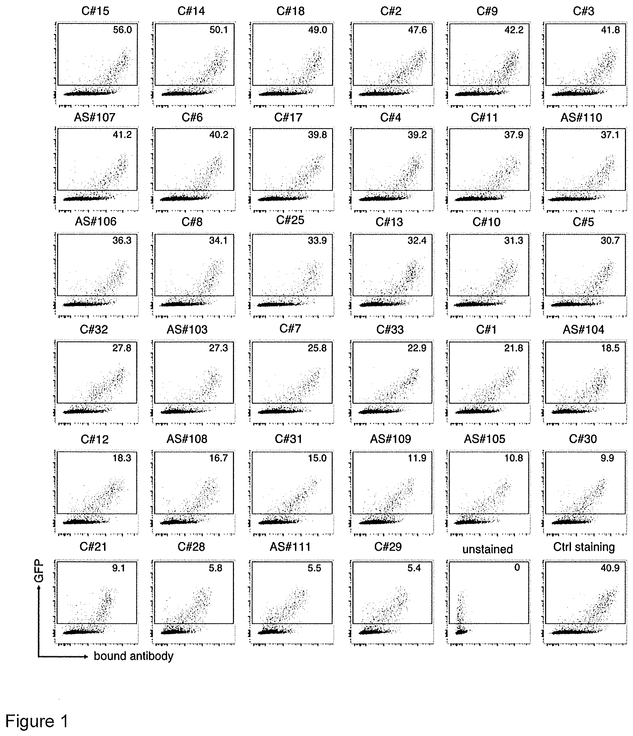

**Figure 1** shows the results of the analysis of hcAbs binding to CD229-transfected HEK293-T cells by flow cytometry. HEK293-T cells were co-transfected with expression vectors for green fluorescent protein (GFP) and human CD229. 24h after transfection, cells were incubated with HEK cell supernatants containing the indicated rabbit or human IgG1 hcAbs. Bound antibodies were detected with PE-conjugated anti-rabbit or anti-human IgG polyclonal antibody.

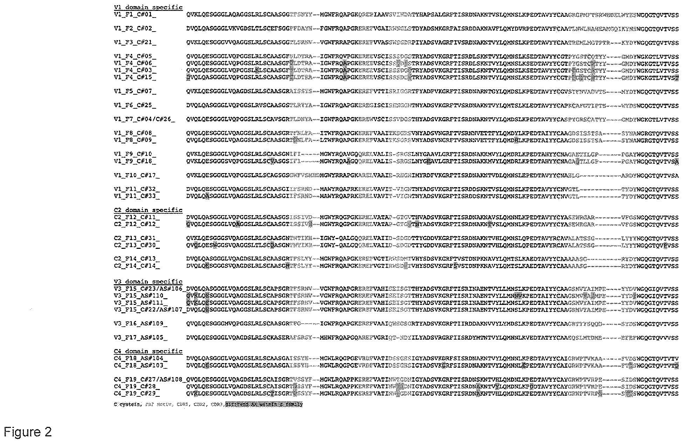

**Figure 2** shows the results of the amino acid sequence alignment of the VHHs of the identified CD229-specific hcAbs. The amino acid sequences are sorted according to their binding specificity to one of the four domains (V1, C2, V3, C4) of human CD229 and their respective family. Sequences of the three complementary regions (CDR) are indicated: CDR1 in red CDR2 in green and CDR2 in blue. The hall mark residues of the VHH domain are marked in magenta. Cysteines are indicated in yellow. Amino acid varying within a family are indicated in cyan.

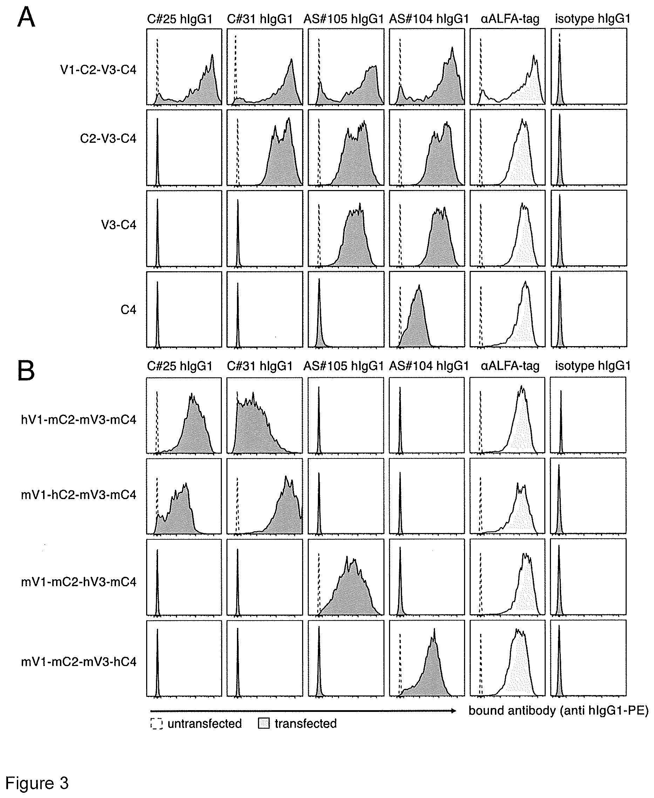

**Figure 3** shows the results of the analyses of hcAbs binding to CD229 domains by flow cytometry. Binding to truncated (A) and chimeric (B) CD229 were analyzed. Bound hcAbs are indicated in dark and light grey. Unstained controls are indicated by dashed lines.

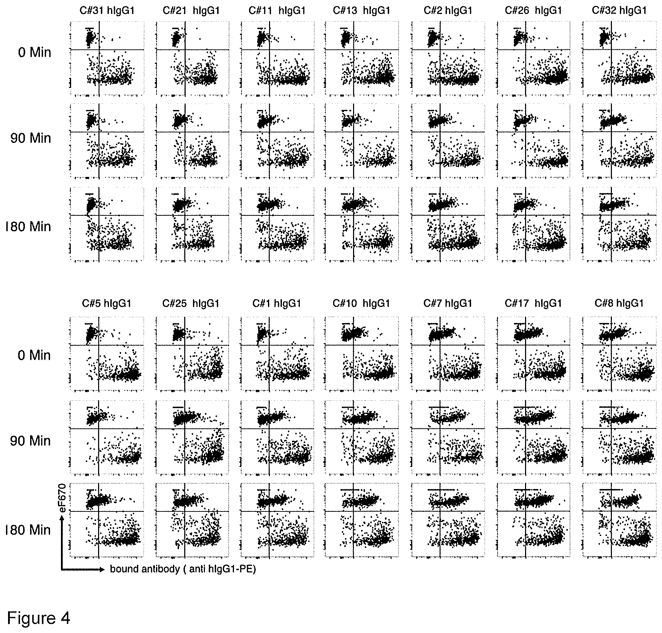

**Figure 4** shows the results of the relative dissociation analysis of CD229-specific hcAbs. Dye eFluor670 labelled CD229 expressing HEK293-T cells functioned as a sink for hcAbs dissociated from CD229 expressing HEK293-T cells and were detected by human IgG1-specific, PE-conjugated secondary antibody. PE and eFluor670 fluorescence signals were detected directly, 90 min and 180 min after addition of eFluor670 labelled cells.

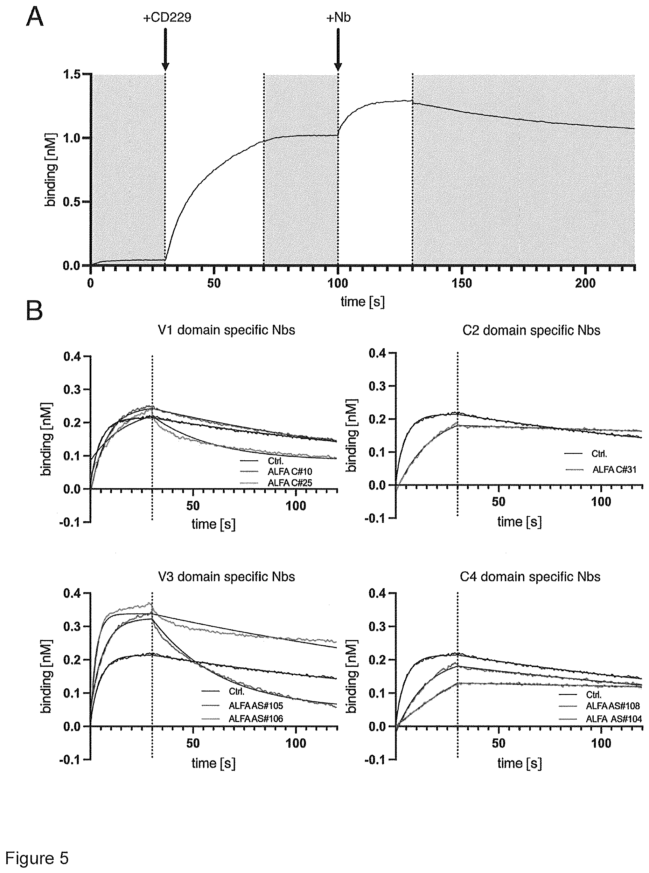

**Figure 5** shows the results of the Biolayer interferometry (BLI) measurement. The sensorgrams demonstrate binding of ALFA-tagged CD229-specific Nbs to immobilized CD299 (A) and individual binding of the CD229-specific Nbs ALFA _C#25, ALFA _C#31, ALFA_C#10, ALFA_AS#104, ALFA_AS#105, ALFA_AS#106 and ALFA_AS#108.

### EXAMPLES

The present invention is further described in more detail by the following examples which are only provided for illustrating the invention and which are not to be construed as limiting the scope of the invention. The following material and methods were used in the Examples.

### 1. Immunizations

1.1 The open reading frames of human CD229 (gene ID 4063) and mouse CD229 (gene ID 17085) were cloned into the pCMV-SPORT6 expression vector via EcoRI and NotI restriction sites. The ectodomain of CD229 was expressed as a recombinant protein with a C-terminal His6x tag or with the hinge, CH2 and CH3 domains of Ilama IgG2b in transiently transfected HEK-6E-cells and purified by immobilized metal affinity chromatography or by protein-A chromatography.
1.2 Two alpacas were immunized by a DNA-prime protein boost strategy as described previously (see Koch-Nolte et al. 2007). The humoral immune response was monitored in serially diluted serum using HEK293-T cells transfected with the same plasmids used for immunization by and fluoromchrome-conjugated Ilama IgG-specific secondary antibodies by flow cytometry. Animals were bled 9 days after the 4^{th} DNA immunization and four days after the 1^{st} and 2^{nd} protein boost.

### 2. Phage display library construction

2.1 Mononuclear cells were isolated from 100 ml of blood by Ficoll-Paque^{™} (GE Healthcare, Chalfont St Giles, UK) gradient centrifugation. RNA purified from these cells by NucleoSpin RNA Mini Kit (Macherey-Nagel, Düren, Germany) was subjected to cDNA synthesis with Ilama IgG-specific primers (Table 1). The nanobody coding region was amplified by PCR with nanobody-specific primers.
2.2 PCR products were purified from agarose gels, digested with SfiI and NotI (NEB, Ipswich, MA) and cloned into the pHEN2 phagemid vector downstream of the PelB-leader peptide and upstream of the gp3 M13 phage protein (see Koch-Nolte et al. 2007). Transformation into XL1-Blue *E. coli* (Stratagene, La Jolla, CA) yielded libraries with sizes of 10⁶-10⁷ clones.
2.3 Phage particles were precipitated with polyethylene glycol from culture supernatants of transformants infected with an excess of KM-13 helper phage (GE Healthcare, Chalfont St Giles, UK). Panning of specific phage was performed by binding to HEK293-T cells stably transfected with human CD229 or by binding to ELISA plates coated with recombinant His6x-tagged extracellular domain of human CD229 (Acro BIOSYSTEMS, Newark, NJ). Phage particles (1 × 10¹¹) were incubated with either human CD229-transfectetd HEK cells (10⁶ cells in 1 mL DMEM medium containing 10% FCS) or with recombinant human CD229 (100 ng/well) in PBS containing 2% BSA with agitation. Following extensive washing, bound phages were eluted from transfected cells by trypsinization.
2.4 Eluted phages were used to infect TG1 *E. coli* cells (Lucigen, WI). Plasmid DNA was isolated from single colonies and subjected to sequence analyses using pHEN2-specific forward and reverse primers (Table 1).

**Table 1: Primers used for cloning**

| Primer | Sequence |
|---|---|
| CMV forward - SEQ ID NO:138 | 5'-cgcaaatgggcggtaggcgtg-3' |
| BGH reverse - SEQ ID NO:139 | 5'-tagaaggcacagtcgagg-3' |
| SHF (mu) forward - SEQ ID NO:140 | 5'-tcgcggcccagccggccatggcgcaggtsmarctgcaggagtcwgg-3' |
| LHF (mu) forward - SEQ ID NO:141 | 5'-tcgcggcccagccggccatggccgatgtgcagctgcaggmgtcwggrggagg-3' |
| reverse IgG2b_NotI_TE - SEQ ID NO:142 | 5'-aggattgggttgtggtgcggccgctggttgtggttttggtgtcttgggttc-3' |
| reverse IgG2c_rev_new - SEQ ID NO: 143 | 5'-cctgggcatttgggagcggccgcgctggggtcttcgctgtggtg-3' |

### 3. Production of nanobodies and hcAbs

3.1 The coding region of selected nanobodies (Nbs) was subcloned via NcoI and NotI restriction sites into the pCSE2.5 vector (Jäger et al. 2013) upstream of either an ALFA tag, FLAG tag or the hinge and Fc-domains of rabbit IgG or human IgG1 (Eden et al. 2017). Recombinant ALFA or FLAG-tagged Nbs and rabbit IgG or human IgG1 heavy chain antibodies (hcAbs) were expressed in transiently transfected HEK293-6E cells cultivated in serum-free medium. Five to six days post transfection, supernatants were harvested and cleared by centrifugation.
3.2 Nanobodies and hcAbs in cell supernatants were quantified by SDS-PAGE and Coomassie staining relative to marker proteins of known quantities.

### 4. Flow cytometry

4.1 For binding analyses, untransfected HEK293-T cells and HEK293-T_hCD229-GFP cells stably transfected with human CD229 and GFP were incubated for 60 min with HEK293-6E cell supernatants containing rabbit or human IgG hcAbs (diluted 1:20 in PBS/0.2% BSA). Following washing with PBS/0.2% BSA, bound antibodies were detected with PE-conjugated goat anti-rabbit or donkey anti-human IgG polyclonal antibody (711-116-152, 709-116-149 Jackson Immuno Research, Dianova, Hamburg).
4.2 For epitope analyses, cells were pre-incubated with excess human IgG1 hcAbs (10 µl HEK293-6E cell supernatant/100 µl PBS/0.2% BSA) for 30 min at RT before addition of rabbit IgG hcAbs (1 µl HEK293-6E cell supernatant/100 µl PBS/0.1% BSA) and further incubation for 20 min at RT and staining with AF647-conjugated anti-rabbit IgG polyclonal antibody (711-116-152, Jackson Imuno Research, Dianova, Hamburg). Cells were washed and analyzed by flow cytometry on a BD-FACS Celesta. Data were analyzed using the FlowJo software (Treestar).
4.3 For dissociation analyses, two separate aliquots of HEK293-T cells transiently transfected with human CD229 and GFP were incubated either with Cell Proliferation Dye eFluor 450 (eBioscience) or with hcAbs for 20 min at RT. Cells were washed three times, mixed and further incubated before staining of bound hcAbs with PE-conjugated donkey anti-human IgG-specific secondary antibody (Dianova, Hamburg). The dissociation of hcAbs from the target cells and association with the eFluor 450 labeled cells was analyzed by flow cytometry using the FlowJo software (Treestar).
4.4 Affinity assays were performed by BLI-technology using a fortéBIO BLItz instrument at 20°C with running buffer (PBS, 0.002% (v/v) Tween-20). Penta-His sensors (Octet HISIK Biosensors, SATORIUS, Göttingen, Germany) were hydrated in the running buffer and loaded for 40 sec with His6x-tagged human CD229 (Acro-Biosystems, DE, United States). After washing, HEK-6E supernatants of ALFA-tagged nanobodies were allowed to associate for 30 sec on immobilized CD229, followed by dissociation for 90 sec. Curve fitting and affinity calculations were performed using Graph Pad Prism version 10.0.3 (GraphPad Software, CA, USA).

### Example 1: Induction of CD229-specific hcAbs

Two alpacas were immunized with CD229 for the induction of hcAbs. In particular, alpacas were immunized and boosted with CD229 expression vectors by ballistic immunization essentially as described previously (Koch-Nolte et al 2007). Each alpaca received four DNA immunizations with administration intervals of two weeks. Each dose consisted of 12 shots of plasmid-conjugated gold particles (1 µg of DNA conjugated onto 0.5 mg gold particles per shot) applied with a pressure setting at 600 psi into the skin. Three weeks after the final genetic immunization, two subsequent protein boosts were performed. At regular intervals, blood samples were collected to monitor the induction of the humoral immune response over time. For the isolation of B-cells, blood was collected from the animals nine days after the 4^{th} DNA immunization and four days after the 1^{st} and 2^{nd} protein boost. Peripheral blood mononuclear cells were prepared from blood samples using Ficoll-Paque^{™} gradient centrifugation.

### Example 2: Selection of VHHs from phage display libraries

To create a phage display library RNA was isolated from the peripheral blood lymphocytes of alpacas after the last injection using the NucleoSpin RNA Mini Kit (Macherey-Nagel, Düren, Germany) and converted into cDNA by reverse transcription. For reverse transcription a primer was used that binds to the CH2-domain of alpaca IgG2b and IgG2c isotypes. The coding region of the VHH was amplified by PCR and the VHH repertoire was cloned via specific restriction sites into the pHEN2 phagemid vector downstream of the PelB-leader peptide and upstream of the gp3 M13 phage protein. The ligation products were then transformed into XL1-Blue *E. coli* (Stratagene, La Jolla, CA) to create a phage library expressing VHH fragments. Phages were prepared according to standard protocols.

Selection of CD229-specific VHH from the immune phage libraries was performed using cell selections. Specific phages were enriched by binding to HEK293-T cells stably transfected with human CD229 or by binding to ELISA plates coated with recombinant His6x-tagged extracellular domain of human CD229. Phage particles (1 x 10¹¹) were incubated either with human CD229-transfected HEK cells or with Maxisorb plates coated with recombinant human CD229. Bound phages were eluted from transfected cells or recombinant protein and used to infect TG1 *E. coli* cells (Lucigen, WI). Plasmid DNA was isolated from single colonies and subjected to sequence analyses. Clones that were found more than once and clones with the same framework and a highly similar CDR3 carrying with or without additional amino acid substitutions were defined as a clone family.

### Example 3: Recombinant expression of VHH in HEK293-6E cells

Representative VHHs of the above identified clones were expressed as nanobodies (Nbs) either with an ALFA tag or a FLAG tag or as heavy chain antibodies (hcAbs) comprising the hinge, CH2 and CH3 domains of rabbit IgG or human IgG in HEK293-6E cells. The Nbs were purified by immobilized metal affinity chromatography, the hcAbs were purified by Protein A affinity chromatography. SDS-PAGE analysis confirmed the purity, integrity and concentration of the purified Nbs and hcAbs (not shown).

### Example 4: Analysis of hcAbs binding to CD229-transfected HEK293 cells by flow cytometry

The binding specificities of the purified hcAbs to CD229-transfected HEK293-T cells were determined by flow cytometry using HEK cells transiently co-transfected with expression vectors for GFP and either rabbit CD229 or human CD229. Cells were incubated with rabbit or human IgG hcAb containing HEK293-6E cell supernatants for 60 min at 4°C. After washing, cells were incubated with PE-conjugated goat anti-rabbit or donkey ant-human IgG polyclonal antibody (711-116-152, 709-116-149, Dianova, Hamburg). The cells were resuspended and analyzed by flow cytometry. Data evaluation was conducted using the FlowJo software (Treestar, Stanford, US).

Results: The results are shown in Figure 1. It can be seen that 34 rabbit and human hcAbs showed specific binding to human CD229 and GFP co-transfected HEK293-T cells. The control staining was performed with an irrelevant rabbit IgG hcAb and did show little to no reactivity. Figure 2 shows an amino acid sequence alignment of the VHH domains of the 34 selected CD229-specific hcAbs. The amino acid sequences are sorted according to their determined binding specificity to the four domains (V1, C2, V3, C4) of CD229 and family.

### Example 5: Epitope analysis

To evaluate the epitopes on the CD229 protein recognized by different VHHs, HEK293-T cells were transiently co-transfected with expression vectors for human CD229 and GFP and analyzed by flow cytometry. Cells were pre-incubated with excess human IgG1 hcAbs for 30 min at 4°C to block specific epitopes before addition of rabbit IgG hcAbs as detectors and further incubation for 15 min at 4°C. Cells were washed and bound antibodies were detected with AF647-conjugated anti-rabbit IgG polyclonal antibody. Cells were washed and analyzed by flow cytometry on a BD-FACS Celesta. Data were analyzed using the FlowJo software (Treestar). Control stainings were performed in the absence of a pre-blocking antibody.

Results: The results of the epitope binning analyses with human CD229-expressing cells are summarized in Table 2. Human IgG hcAb C#5 (family 4) blocked the binding of rabbit IgG hcAb C#5 to human CD229-transfected HEK cells (designated epitope 1). In contrast, human hcAb C#5 did not block the binding of any of the rabbit IgG hcAb analyzed of families 6, 13, 17, 15 and 18 indicating that these hcAbs recognize distinct epitopes on human CD229. The results show that the selected hcAbs bin to six non-overlapping epitopes on CD229.

**Table 2: Epitope assignment of human CD229-specific hcAbs. The human IgG hcAbs used for blocking are indicated on top. Rabbit hcAbs used for detection are indicated in the column "Name". Numbers indicate percent inhibition of binding. Epitope indicates binding to distinct epitopes.**

| | | | | Blocking hcAb | | | | | |
|---|---|---|---|---|---|---|---|---|---|
| Epitope | Domain | Family | Name | C#5 | C#25 | C#31 | AS# 105 | AS# 106 | AS# 104 |
| 1 | V1 | 4 | C#5 | 100 | 1 | 20 | 71 | 40 | 17 |
| 2 | V1 | 6 | C#25 | -19 | 93 | 2 | 31 | 20 | 21 |
| 3 | C2 | 13 | C#31 | -8 | -51 | 97 | 14 | 5 | 12 |
| 4 | V3 | 17 | AS# 105 | -23 | -26 | 15 | 98 | -18 | -15 |
| 5 | V3 | 15 | AS# 106 | -6 | -29 | 26 | 19 | 99 | 10 |
| 6 | C4 | 18 | AS# 104 | -73 | -91 | -10 | -82 | -81 | 96 |

### Example 6: Analysis of hcAbs binding to the four domains of human CD229

For mapping of the above identified epitopes to one of the four human CD229 domains V1, C2, V3 and C4, the domains were expressed as truncated versions and as chimeric constructs of human and mouse CD229 on the cell surface of transfected HEK293-T cells. Bound hcAbs were detected with PE-conjugated donkey human IgG-specific secondary antibody. Control stainings were performed without use of primary antibodies.

Results: The results of the epitope mapping analysis are shown in Figure 3. HcAbs carrying the above identified epitope-binding VHH domains were analyzed with regard to their binding specificity to truncated (A) and chimeric (B) CD229 expressing HEK293-T cells. Epitopes of C#25, C#31, AS#105 and AS#104 were found to be located on the V1, C2, V3 and C4 domains of CD229, respectively.

### Example 7: Analysis of hcAbs relative dissociation rate from human CD229

For relative dissociation rate analyses, human CD229 and GFP expressing HEK293-T cells were incubated with CD229-specific hcAbs. Cells were monitored for loss of cell-associated fluorescence over time. Bound hcAbs were detected with PE-conjugated donkey anti-human IgG-specific secondary antibody. Cell-associated fluorescence was determined by flow cytometry.

Results: The results are shown in Figure 4. Dye eFluor670 (eF670) labelled CD229 expressing HEK293-T cells were used as a sink for dissociating hcAbs. PE-conjugated antibodies were used for detection of hcAbs stably bound to CD229 expressing cells (not labelled). As shown in Figure 4, signal intensities remained stable indicating comparatively low dissociation rates of the hcAbs from cell-surface CD229.

### Example 7: Analysis of hcAbs binding to soluble CD229 by Biolayer interferometry

The affinity of ALFA-tagged Nb to human CD229 was determined by bioluminescence (BLI) technology using a fortéBIO BLItz instrument. Immobilization of His6x-tagged human CD229 on the Penta-His-biosensor surface was followed by incubation with ALFA-tagged Nbs comprising HEK293-6E supernatants. Association and dissociation were monitored for different CD229-specific Nbs to the immobilized CD229 and compared to the positive control (Ctrl).

Results: The results are shown in Figure 5. The analyzed Nbs ALFA _C#25, ALFA_C#31, ALFA_C#10, ALFA_AS#104, ALFA_AS#105, ALFA _AS#106, ALFA_AS#108 showed a range of affinities. Nbs ALFA_C#106, ALFA_C#10 and ALFA_C#31 were found to have high association and low dissociation rates to human CD229.

### LITERATURE

Eden et al. 2018. Front Immunol. 8:1989. PMID: 29410663
Goueli and Hsiao 2019. PLoS ONE 14: e0220094-19, PMID 31652269
Jäger et al. 2013 BMC Biotechnol. 13:52. PMID: 23802841
Koch-Nolte et al. 2007 FASEB J. 21:3490-8. PMID: 17575259
Remington: The Science and Practice of Pharmacy, Lippincott Williams & Wilkins; 21. ed (2005)

## Claims

1. Antigen-binding polypeptide comprising the CDR1, CDR2 and CDR3 region of a VHH domain of a camelid heavy chain antibody, **characterised in that** the polypeptide specifically binds to CD229, preferably human CD229.

2. Antigen-binding polypeptide according to claim 1, wherein said polypeptide inhibits tumour growth.

3. Antigen-binding polypeptide according to any of claims 1-2, **characterized in that** the polypeptide specifically binds to the V1 domain of CD229, and wherein the polypeptide preferably comprises
(a) the CDR sequences shown in SEQ ID NO:1-3;
(b) the CDR sequences shown in SEQ ID NO:4-6;
(c) the CDR sequences shown in SEQ ID NO:7-9;
(d) the CDR sequences shown in SEQ ID NO:10-12;
(e) the CDR sequences shown in SEQ ID NO:13-15;
(f) the CDR sequences shown in SEQ ID NO:16-18;
(g) the CDR sequences shown in SEQ ID NO:19-21;
(h) the CDR sequences shown in SEQ ID NO:22-24;
(i) the CDR sequences shown in SEQ ID NO:25-27;
(j) the CDR sequences shown in SEQ ID NO:28-30;
(k) the CDR sequences shown in SEQ ID NO:31-33;
(l) the CDR sequences shown in SEQ ID NO:34-36;
(m) the CDR sequences shown in SEQ ID NO:37-39;
(n) the CDR sequences shown in SEQ ID NO:40-42;
(o) the CDR sequences shown in SEQ ID NO:43-45;
(p) the CDR sequences shown in SEQ ID NO:46-48;
(q) the CDR sequences shown in SEQ ID NO:49-51;
or CDR sequences which differ from the CDR sequences shown in (a)-(q) in not more than one amino acid per CDR region.

4. Antigen-binding polypeptide according to any of claims 1-2, **characterized in that** the polypeptide specifically binds to the C2 domain of CD229, and wherein the polypeptide preferably comprises
(a) the CDR sequences shown in SEQ ID NO:52-54;
(b) the CDR sequences shown in SEQ ID NO:55-57;
(c) the CDR sequences shown in SEQ ID NO:58-60;
(d) the CDR sequences shown in SEQ ID NO:61-63;
(e) the CDR sequences shown in SEQ ID NO:64-66;
(f) the CDR sequences shown in SEQ ID NO:67-69;
or CDR sequences which differ from the CDR sequences shown in (a)-(f) in not more than one amino acid per CDR region.

5. Antigen-binding polypeptide according to any of claims 1-2, **characterized in that** the polypeptide specifically binds to the V3 domain of CD229, and wherein the polypeptide preferably comprises
(a) the CDR sequences shown in SEQ ID NO:70-72;
(b) the CDR sequences shown in SEQ ID NO:73-75;
(c) the CDR sequences shown in SEQ ID NO:76-78;
(d) the CDR sequences shown in SEQ ID NO:79-81,
(e) the CDR sequences shown in SEQ ID NO:82-84;
(f) the CDR sequences shown in SEQ ID NO:85-87;
or CDR sequences which differ from the CDR sequences shown in (a)-(f) in not more than one amino acid per CDR region.

6. Antigen-binding polypeptide according to any of claims 1-2, **characterized in that** the polypeptide specifically binds to the C4 domain of CD229, and wherein the polypeptide preferably comprises
(a) the CDR sequences shown in SEQ ID NO:88-90;
(b) the CDR sequences shown in SEQ ID NO:91-93;
(c) the CDR sequences shown in SEQ ID NO:94-96;
(d) the CDR sequences shown in SEQ ID NO:97-99,
(e) the CDR sequences shown in SEQ ID NO:100-102,
or CDR sequences which differ from the CDR sequences shown in (a)-(e) in not more than one amino acid per CDR region.

7. Antigen-binding polypeptide according to any of claims 1-2, **characterized in that**
(i) the polypeptide specifically binds to the V1 domain of CD229 and comprises an amino acid sequence selected from the group consisting of:
(i-a) the amino acid sequences of SEQ ID NO:103-119; and
(i-b) an amino acid sequence having at least 80% or more sequence identity to one of the amino acid sequences of SEQ ID NO:103-119; or
(ii) the polypeptide specifically binds to the C2 domain of CD229 comprises an amino acid sequence selected from the group consisting of:
(ii-a) the amino acid sequences of SEQ ID NO:120-125; and
(ii-b) an amino acid sequence having at least 80% or more sequence identity to one of the amino acid sequences of SEQ ID NO:120-125; or
(iii) the polypeptide specifically binds to the V3 domain of CD229 comprises an amino acid sequence selected from the group consisting of:
(iii-a) the amino acid sequences of SEQ ID NO:126-131; and
(iii-b) an amino acid sequence having at least 80% or more sequence identity to one of the amino acid sequences of SEQ ID NO:126-131; or
(iv) the polypeptide specifically binds to the C4 domain of CD229 comprises an amino acid sequence selected from the group consisting of:
(iv-a) the amino acid sequences of SEQ ID NO:132-136; and
(iv-b) an amino acid sequence having at least 80% or more sequence identity to one of the amino acid sequences of SEQ ID NO:132-136.

8. Fusion protein comprising an antigen-binding polypeptide according to any one of claims 1-7, **characterized in that** the fusion protein comprises a VHH domain directed against CD38, CD269 or CD138.

9. Conjugate comprising an antigen-binding polypeptide according to any one of claims 1-7 or a fusion protein according to claim 8 coupled to a cytotoxic moiety.

10. Pharmaceutical composition comprising an antigen-binding polypeptide according to any one of claims 1-7, a fusion protein according to claim 8 or a conjugate according to claim 9.

11. Antigen-binding polypeptide of any one of claims 1-7, fusion protein of claim 8, conjugate of claim 9 or pharmaceutical composition of claim 10 for use in a method of treating a disease which is **characterized by** a pathologically enhanced level of CD229 activity, preferably a hyperproliferative disease, and more preferably a hyperproliferative disease selected from B-cell lymphoma, multiple myeloma (MM), marginal-zone lymphomas (MZL), chronic lymphocyte lymphoma (CLL), follicular lymphoma (FL), mantle-cell lymphoma (MCL), diffuse large B cell lymphoma, or a CD229-expressing solid tumour.

12. Nucleic acid molecule encoding an antigen-binding polypeptide according to any one of claims 1-7.

13. Expression vector comprising a nucleic acid molecule according to claim 12.

14. Host cell comprising a nucleic acid molecule according to claim 12 or an expression vector according to claim 13.

15. A method for the recombinant production of an antigen-binding polypeptide according to any one of claims 1-7, comprising:
(a) culturing a host cell according to claim 14 under conditions which allow expression of a nucleic acid molecule according to claim 12; and
(b) isolating the antigen-binding polypeptide from the culture.

16. A method for the purification and/or concentration of CD229 and/or CD229-expressing cells in a biological sample, which comprises
(a) contacting an antigen-binding polypeptide according to any one of claims 1-7 with the sample under conditions that allow the formation of complexes of the polypeptide and CD229; and
(b) separating the complexes are from the sample.

17. A method for the detection of CD229 and/or CD229-expressing cells in a biological sample, comprising
(a) contacting an antigen-binding polypeptide according to any one of claims 1-7 with the sample under conditions that allow the formation of complexes of the polypeptide and CD229; and
(b) detecting the complexes in the sample.

18. A method for the diagnosis of a disease **characterised by** increased CD229 expression comprising
(a) contacting an antigen-binding polypeptide according to any one of claims 1-7 with a biological sample from a patient under conditions that allow the formation of complexes of the polypeptide and CD229; and
(b) detecting complexes of the polypeptide and CD229/CD229-expressing cells;
wherein detection of the complexes in step (b) indicates that the patient has a disease **characterised by** increased CD229 expression.

19. Method according to claim 18, wherein the method comprises the step of comparing the amount of complexes detected in step (b) with an amount of a control.

20. Method according to claim 18 or 19, **characterized in that** the disease is hyperproliferative disease, preferably selected from the group of B-cell lymphoma, multiple myeloma (MM), marginal-zone lymphomas (MZL), chronic lymphocyte lymphoma (CLL), follicular lymphoma (FL), mantle-cell lymphoma (MCL), diffuse large B cell lymphoma, or a CD229-expressing solid tumour.

21. Kit for detecting CD229 and/or CD229-expressing cells, **characterised in that** the kit comprises an antigen-binding polypeptide according to any one of claims 1-7.
